# EUROPEAN PATENT APPLICATION

(11) **EP 2 572 582 A1**
(43) Date of publication of application: **27.03.2013**
(21) Application number: 11783114.9
(22) Date of filing: 15.04.2011
(51) Int. Cl.: A01N 65/22, A61K 8/92, A61Q 17/00

(54) **FORMULATION FOR PRESERVING COSMETIC PRODUCTS**

(30) Priority: 19.05.2010 ES 201030744
(71) Applicant: Novejarque Conde, José Antonio, 46011 Valencia (ES)
(72) Inventor: Novejarque Conde, José Antonio, 46011 Valencia (ES)
(74) Representative: Ungria López, Javier
(86) International application number: PCT/ES2011/070270
(87) International publication number: WO 2011/144782

(57) **Abstract**

Formulation for preserving cosmetic products. The present invention relates to a formulation for preserving cosmetic products characterised in that it comprises: a) at least one first natural ingredient selected from a group comprising calendula oil (Calendula officinalis), cinnamon oil (Cinnamomum verum), clove oil (Oleum caryophylli), oregano oil (Origanum vulgare), thyme oil (Thymus vulgaris), Siberian pine oil (Pinus sibirica) and rosemary oil (Rosmarinus officinalis), together with any combination thereof; b) at least one second natural ingredient selected from a group consisting of organic rosehip oil (Rosa eglanteria), organic coconut oil, cardamom oil (Elettaria cardamomum), propolis, organic argan oil (Argania spinosa) and benzyl carbinol, together with any combination thereof. The object of the invention is moreover a procedure for the obtainment of said formulation and the use thereof for the preparation of cosmetic products.

## Description

### Technical Field

The present invention falls within the cosmetic field. Specifically, it relates to a new formulation for preserving cosmetic products.

### State of the art prior to the invention

In recent years, due to the growing demand from consumers for high quality products, that at the same time, are also environmentally friendly, new ecological products have emerged on the market, among which, cosmetics based on natural components can be found.

While in the European Union it is possible to find specific legislation relating to "bio" foods, the same does not apply to the case of cosmetic products, such that, currently, there does not exist any European regulation that outlines the requirements that this type of cosmetic products should meet, in terms of permitted substances, proportion of ingredients from natural and organic origin, labeling regulations, etc. Given the absence of legislation, in order to guarantee the natural or ecological nature of the cosmetics, the manufacturers of said products are subjected to the criteria established by private certifying companies.

Among these criteria, are those related to the preservatives used in the formulation of the cosmetic products with the objective of preventing microbial contamination during their manufacturing, their storing and their daily use by the end-consumer. Generally, the preservatives are defined as chemical substances having an antimicrobial activity, which are incorporated to the cosmetics in a very small concentration (between 0.0005 and 1% of active substance) during their manufacturing process.

Upon selecting the preservatives used in the formulation of the cosmetic products, it is desirable that these comply with a number of characteristics, among which should be noted, for example, their spectrum of antimicrobial activity, solubility in water, stability in extreme pH and temperature conditions, cost and compatibility with the rest of the ingredients in the formulation.

Up until now, the preservatives most used in the state of the art, have been parabens, most of them combined with phenoxyethanol and formaldehyde donors.

W02009070736, for example, describes a method for preventing microbiological contamination in a cosmetic or pharmaceutical formulation, which comprises adding an effective amount of at least one hydroxamic acid, a salt and/or a derivative thereof.

EP1486200 describes a cosmetic or dermatological formulation that contains potassium sorbate, as well as stabilizers selected from microcrystalline cellulose and/or talc, or is based on an emulsion that contains lipids, having a surface tension, of at least 10 mN/m, as stabilizers.

Therefore, the object of this invention is to introduce a new alternative to the previous formulations for preserving cosmetic products, characterized in that they are based on ingredients from natural origin.

### Description of the invention

Thereby, the present invention relates to a new formulation for preserving cosmetics products, characterized in that it comprises:
a) at least one first natural ingredient selected from a group consisting of: calendula oil (*Calendula officinalis*), cinnamon oil (*Cinnamomun verum*), clove oil (*Oleum caryophylli*), oregano oil (*Origanum vulgare*), thyme oil (*Thymus vulgaris*), Siberian cedar oil (*Pinus sibirica*) and rosemary oil (*Rosmarinus officinalis*), as well as any combination thereof;
b) at least one second natural ingredient selected from a group consisting of organic rosehip oil (*Rose canina* or *Rosa eglanteria*), organic coconut oil, cardamom oil *(Elettaria cardamomum*), propolis, organic argan oil (*Argania spinosa*) and benzyl carbinol, as well as any combination thereof.

Preferably, each one of the ingredients of the formulation can be found in a weight percentage between 0.01 and 80% with respect to the total weight of the formulation.

In a particular embodiment of the invention, the preserving formulation can also, preferably, but non-limiting, include at least one chemical product, selected from a group consisting in: benzyl alcohol, sodium benzoate, potassium sorbate, and benzoic acid, as well as any combination thereof. Preferably, the percentage of said chemical products with respect to the total of the formulation can be between 0 and 5%w/w.

Furthermore, in order to provide a suitable vehicle so the incorporation of the formulation, objet of the invention, is carried out in a homogenous manner, it may further comprise at least one vehicle selected from a group consisting, in a preferred but not limiting manner, of rosemary essential oil, organic grape seed oil, or organic argan oil. Preferably, said vehicle may be present in the formulation, in a percentage between 10 and 40%w/w.

The combination of the above compounds allows for obtaining a synergistic effect, giving rise to a formulation with optimum preserving characteristics.

Generally, the elaboration procedure of the formulation, object of the invention, can comprise the following stages:
a) the weighing and mixing of at least one first natural ingredient selected from a group consisting of: calendula oil, cinnamon oil, clove oil, oregano oil, thyme oil, Siberian cedar oil and rosemary oil, as well as any combination thereof, giving rise to phase A of the mixture.
b) the weighing and mixing of at least one second natural ingredient selected from a group consisting of organic rosehip oil, organic coconut oil, cardamom oil, propolis, organic argon oil and benzyl carbinol, as well as any combination thereof, giving rise to phase B of the mixture.
c) The mixing of phase A and phase B, giving rise to the formulation object of the invention.

In a preferred embodiment of the invention, the procedure can, in turn, comprise of a prior quality control stage of the ingredients used in the procedure.

Insofar as the different ingredients corresponding to phase A are selected and mixed, they are introduced in at least one first mixing tank. In said first mixing tank, the different ingredients are subjected to continuous stirring, preferably, at a temperature between 20 and 45 °C and during a time ranging from 15 to 25 minutes. Once this time has elapsed, the homogeneous mixture obtained is left to stand for a time ranging, generally, between 15 and 30 minutes until a temperature of approximately 25 °C is reached.

In a preferred embodiment of the invention, simultaneously with the previous stage, in at least one second different mixing tank and separate from the first mixing tank, the addition of the different ingredients selected from the group corresponding to phase B of the mixture will be carried out, once weighed. In said second mixing tank, the homogeneous mixing of said ingredients takes place for a period of time, preferably, between 15 and 25 minutes at a preferred temperature, between 20 and 45 °C. Once this stage has been completed, phase A, as well as phase B of the mixture, are pumped into at least one third mixing tank, where they are subjected to stirring, preferably, at a room temperature of approximately, 25 °C and for a time ranging between 10 and 20 minutes, until a homogeneous mixture is obtained, corresponding to the final formulation, which is then left to stand for a time of, approximately, 10 minutes.

In a preferred embodiment of the invention, the preparation and mixing of the different ingredients of the process, take place in cleanrooms, specifically designed for that purpose, in compliance with the regulation UNE-EN ISO 14644.

Similarly, in a particular embodiment of the invention, the described procedure can, in turn, comprise an additional packaging stage of the formulation, as well as a storing stage prior to its distribution.

Preferably, the facility for carrying out the above method is characterized in that it comprises at least three separate mixing tanks, preferably, of stainless steel and with a stirring mechanism, whose capacity depends on the desired amount of end product obtained. In a particularly preferred embodiment of the invention, the facility is characterized in that it comprises at least two intermediate containers of 500L and at least one final container of 1000L of capacity, preferably, made of stainless steel and having a stirring mechanism. Preferably, said stirring is carried out by using stirrers, preferably made of stainless steel and with a rotation rate ranging, preferably, between 200 and 500 rpm.

Similarly, a further object of this invention is the use of a preserving formulation as it has been previously described, for the production of at least one cosmetic product, as well as the cosmetic product itself, characterized in that it comprises said formulation, preferably, in a weight percentage comprised between 0.2 and 3% of the total cosmetic product.

In a preferred embodiment of the invention, the preserving formulation is characterized in that it is intended for its incorporation, preferably, to cosmetics that comply with the ECOCERT regulations, although it may also be used in conventional cosmetics.

### Preferred embodiment of the invention

Next, by way of non-limitative example, a particular embodiment, object of the present invention, is described. In this case, the formulation for preserving cosmetic products was prepared based on the mixture of two phases (phase A and phase B), with a composition weight with respect to the total weight of the final mixture as follows:

| Phase A | % |
|---|---|
| Calendula oil | 4 |
| Cinnamon oil | 5 |
| Clove oil | 5 |
| Oregano oil | 7 |
| Thyme oil | 7 |
| Siberian pine oil | 10 |
| Organic rosemary oil | 1 |
| | |

| Phase B | % |
|---|---|
| Organic rosehip oil | 10 |
| Organic coconut oil | 1 |
| Cardamom oil | 4 |
| Propolis | 7 |
| Benzyl carbinol | 1 |
| Organic argan oil | 38 |

Both phase A and phase B were prepared in containers separate from the mixture with a stirring mechanism, being subsequently mixed in a third container to obtain the preserving formulation of the invention.

Said formulation was used in the manufacturing of a cosmetic product in a percentage of 2% in weight with respect to the total weight.

## Claims

1. Formulation for preserving cosmetic products **characterized in that** it comprises:
a) at least one first natural ingredient selected from a group consisting of calendula oil *(Calendula officinalis),* cinnamon oil *(Cinnamomun verum),* clove oil *(Oleum caryophylli),* oregano oil *(Origanum vulgare),* thyme oil *(Thymus vulgaris),* Siberian cedar oil *(Pinus sibirica),* rosemary oil *(Rosmarinus officinalis),* and any combination thereof;
b) at least one second natural ingredient selected from a group consisting of organic rosehip oil *(Rosa eglanteria),* organic coconut oil, cardamom oil (*Elettaria cardamomum*), propolis, organic argan oil (*Argania spinosa*), benzyl carbinol, and any combination thereof.

2. Formulation, according to claim 1, **characterized in that** it comprises, additionally, at least one chemical product selected from a group consisting of benzyl alcohol, sodium benzoate, potassium sorbate, benzoic acid, and any combination thereof.

3. Formulation, according to claim 1 or 2, **characterized in that** it comprises, additionally, at least one vehicle selected from a group consisting of rosemary essential oil, organic grape seed oil, organic argan oil, and any combination thereof.

4. Process for obtaining the formulation according to any one of the previous claims, **characterized in that** it comprises the following stages:
a) the weighing and mixing of at least one first natural ingredient selected from a group consisting of calendula oil, cinnamon oil, clove oil, oregano oil, thyme oil, Siberian cedar oil, rosemary oil, and any combination thereof, giving rise to phase A of the mixture;
b) the weighing and mixing of at least one second natural ingredient selected from a group consisting of organic rosehip oil, organic coconut oil, cardamom oil, propolis, organic argon oil, benzyl carbinol, and any combination thereof, giving rise to phase B of the mixture;
c) the mixing of phase A and phase B.

5. Process, according to claim 4, wherein the mixture corresponding to each of the stages (a) and (b) is carried out during a time ranging between 15 and 25 minutes.

6. Process, according to claim 4 or 5, wherein the mixture corresponding to each of the stages (a) and (b) is carried out at a temperature ranging between 20 and 45° C.

7. Process, according to any one of claims 4 to 6, wherein the mixture from phase A and phase B corresponding to stage (c), is carried out during a time ranging between 10 and 20 minutes.

8. Use of the formulation, according to any one of claims 1 to 3, for the preparation of at least one cosmetic product.

9. Cosmetic product **characterized in that** it comprises a formulation according to any one of claims 1 to 3.
